# EUROPÄISCHE PATENTSCHRIFT

(11) **EP 2 760 517 B1**
(45) Veröffentlichungstag und Bekanntmachung des Hinweises auf die Patenterteilung: **02.08.2017**
(21) Anmeldenummer: 12773277.4
(22) Anmeldetag: 27.09.2012
(51) Int. Cl.: A61M 16/16, A61M 16/08

(54) **VERBINDUNGSSYSTEM FÜR ATEMLUFTBEFEUCHTER**
CONNECTION SYSTEM FOR A RESPIRATORY HUMIDIFIER
SYSTÈME DE RACCORDEMENT POUR HUMIDIFICATEUR D'AIR INHALÉ

(30) Priorität: 01.10.2011 DE 102011054134
(43) Veröffentlichungstag der Anmeldung: 06.08.2014
(73) Patentinhaber: Hamilton Medical AG, 7402 Bonaduz (CH)
(72) Erfinder: BÜCHI, Rudolf, 7000 Chur (CH); FREI, Reto, 7402 Bonaduz (CH); MAEDER, Marc, 7208 Malans (CH); GRANZOTTO, Thomas, 7206 Igis (CH); ZOLKOS, Axel, 8864 Reichenburg (CH)
(74) Vertreter: Caspary, Karsten
(86) Internationale Anmeldenummer: PCT/EP2012/069125
(87) Internationale Veröffentlichungsnummer: WO 2013/045575

(56) Entgegenhaltungen:
- EP-A1- 1 369 141
- DE-U1-202007 017 637
- US-A1- 2011 023 874

## Beschreibung

Die vorliegende Erfindung betrifft ein Verbindungssystem zum Anschließen von Beatmungsschläuchen an Atemluftbefeuchter beim Beatmen von Patienten mit Atemgas sowie ein Beatmungssystem umfassend ein Beatmungsgerät, einen Atemluftbefeuchter mit dem Verbindungssystem und ein entsprechendes Beatmungsschlauchsystem.

Bei der maschinellen Beatmung von Patienten, die beispielsweise auf einer Intensivstation liegen, wird der zu beatmende Patient mit Hilfe eines Beatmungsschlauchsystems pneumatisch mit dem Beatmungsgerät verbunden. Da das Atemgas, das dem Patienten zugeführt wird, hinsichtlich Temperatur und Feuchtigkeit den physiologischen Erfordernissen des Patienten angepasst werden muss, wird ein Atemluftbefeuchter in dem Einatem- oder Inspirationsschlauch angeordnet, der das Atemgas anwärmt und anfeuchtet. Der Atemluftbefeuchter weist einen normalerweise mit destilliertem Wasser gefüllten Flüssigkeitsbehälter auf, durch den das Einatemgas geleitet und mit Feuchtigkeit angereichert wird.

Um eine Kondensation von Feuchtigkeit innerhalb des Beatmungsschlauchsystems zu verhindern, sind der Inspirationsschlauch und der Exspirations- oder Ausatemschlauch in üblicher Weise mit einer elektrischen Schlauchheizung versehen, die in Betrieb das durchströmende Ein- oder Ausatemgas erwärmt. Dabei wird beispielsweise eine sich integral innerhalb des Inspirations- oder Exspirationsschlauchs erstreckende Heizdrahtschleife verwendet oder Inspirations- und Exspirationsschlauch sind jeweils mit einer Heizdrahtwendel umwickelt.

Die Regelung der Atemgastemperatur erfolgt üblicher Weise mit Hilfe eines nahe am Patienten angeordneten Temperatursensors, der mittels einer elektrischen Messleitung mit einer Steuereinrichtung, die beispielsweise im Atemluftbefeuchter angeordnet ist, verbunden ist. Es ist daher sinnvoll, die elektrischen Kontaktelemente der elektrischen Leitungen auf den Schlauchverbindern zusammen mit den pneumatischen Verbindungselementen zu gestalten, um die Anzahl der separaten Anschlüsse möglichst weitgehend zu reduzieren.

Pneumatische Schlauchmuffen mit darin integrierten elektrischen Kontaktelementen sind beispielsweise in der EP 1 127 583 A2, der DE 199 58 296 C1, der DE 197 25 875 A1 sowie der US 2003/0059213 A1 beschrieben. Aus der EP 1 369 141 A1 ist ein System bekannt, das die beiden funktionalen Bestandteile des Inspirationsschlauchs mit dem Atemluftbefeuchter verbindet. Dabei weist das Verbindungselement bzw. die Schlauchmuffe einen männlichen Steckerabschnitt am Flüssigkeitsbehälter des Atemluftbefeuchters auf, auf den ein weiblicher Hülsenabschnitt am Ende des Inspirationsschlauchs gesteckt werden kann. Seitlich am Hülsenabschnitt des Inspirationsschlauchs sind elektrische Kontaktelemente angeordnet, die beim Aufstecken des Hülsenabschnitts auf den Steckerabschnitt mit einem weiteren elektrischen Kontakt auf dem Gehäuse des Atemluftbefeuchters in Verbindung gebracht werden, um den elektrischen Kontakt herzustellen.

Nachteilig an dem in der EP 1 369 141 A1 Schlauchverbindungssystem ist, dass es lediglich in einer Richtung, nämlich von oben, auf den Atemluftbefeuchter aufsteckbar ist. Die elektrischen Kontaktelemente auf dem Gehäuse stehen vor dem Verbinden unförmig als eigene Elemente aus dem Gehäuse hervor, was die Optik und Praktikabilität des Atemluftbefeuchters stört. Ein weiterer dadurch entstehender Nachteil ist, dass die Möglichkeiten des Designs für den Atemluftbefeuchter eingeschränkt sind. Sinnvolle Verbesserungen diesbezüglich sind daher bei den bestehenden Geräten kaum möglich.

Es ist daher die Aufgabe der vorliegenden Erfindung, ein Verbindungssystem zum Anschließen eines Beatmungsschlauches an einen Atemluftbefeuchter bereitzustellen, das flexible Anschlussmöglichkeiten für die elektrischen Kontaktelemente bietet und damit eine verbesserte Konstruktion sowie ein ansprechendes Design des Atemluftbefeuchters ermöglicht.

Diese Aufgabe wird durch die Merkmale des Anspruchs 1 gelöst. Vorteilhafte Ausgestaltungen und Ausführungsformen sind in den Unteransprüchen beschrieben.

Erfindungsgemäß wird ein Verbindungssystem zum Anschließen eines Beatmungsschlauches an einen Atemluftbefeuchter bereit gestellt, wobei der Beatmungsschlauch eine elektrische Leitung aufweist und der Atemluftbefeuchter ein Gehäuse und einen Flüssigkeitsbehälter umfasst, mit einem ersten Anschlusselement, das an dem Flüssigkeitsbehälter angeordnet ist, einem ersten elektrischen Kontaktelement, das an dem Gehäuse angeordnet ist, einem zweiten Anschlusselement, das mit dem Beatmungsschlauch verbindbar ist und ein zweites elektrisches Kontaktelement aufweist, das mit der elektrischen Leitung des Beatmungsschlauches verbindbar ist, wobei das erste Anschlusselement und das zweite Anschlusselement entlang einer ersten Verbindungsrichtung derart miteinander verbindbar sind, dass durch Herstellen der pneumatischen Verbindung des Beatmungsschlauches mit dem Flüssigkeitsbehälter auch die elektrische Verbindung des ersten Kontaktelements mit dem zweiten Kontaktelement bewirkt wird. Das Verbindungssystem zeichnet sich dadurch aus, dass die elektrische Verbindung des ersten Kontaktelements mit dem zweiten Kontaktelement in einer von der ersten Verbindungsrichtung abweichenden zweiten Verbindungsrichtung, bevorzugt im Wesentlichen senkrecht zur ersten Verbindungsrichtung herstellbar ist. Dies hat den Vorteil, dass der Flüssigkeitsbehälter mit daran verbundenem Beatmungsschlauch bzw. mit mehreren verbundenen Beatmungsschläuchen z. B. seitlich in das Gehäuse des Atemluftbefeuchters eingeschoben werden kann und dadurch ein kompakter Aufbau des Atemluftbefeuchters ermöglicht wird.

Dabei ist anzumerken, dass die Schlauch- und Verbindungssysteme sowie der Flüssigkeitsbehälter bei in Krankenhäusern verwendeten Beatmungsgeräten als Einmal- bzw. Wegwerfartikel ausgebildet sind, und es deshalb sinnvoll ist, möglichst viele der Verbindungen an den Einmalteilen bereits vorzunehmen, so dass das Bedienpersonal nur noch wenige Verbindungsvorgänge durchzuführen hat. Dadurch wird die Bedienzeit erheblich verkürzt und das Risiko von Fehlverbindungen reduziert. Sollten Flüssigkeitsbehälter und Beatmungsschlauch einmal nicht verbunden sein, so bietet das erfindungsgemäße Verbindungssystem die Möglichkeit, die Schlauchverbindung vor oder nach dem Einsetzen des Flüssigkeitsbehälters in das Gehäuse des Atemluftbefeuchters vorzunehmen. Das seitliche Einschieben des Flüssigkeitsbehälter-Beatmungsschlauchssystem-Kits eröffnet damit neue vorteilhafte Konstruktionsmöglichkeiten des Atemluftbefeuchters.

Bevorzugt sind das erste und das zweite Anschlusselement rohrförmig ausgebildet und weisen einen kreisförmigen Querschnitt auf. Dabei sind das erste und das zweite Anschlusselement in bevorzugter Weise entlang der ersten Verbindungsrichtung axial ineinander einschiebbar. Es versteht sich, dass sich der kreisförmige Querschnitt im Wesentlichen auf die pneumatische Verbindung zwischen Beatmungsschlauch und Feuchtigkeitsbehälter bezieht. Die elektrischen Kontaktelemente sind dann wenigstens abschnittsweise um diesen Querschnitt herum angeordnet. Auch andere Querschnittsformen wie rechteckig, elliptisch oder polygonal sind möglich.

Mit weiterem Vorteil weist das erste Anschlusselement außen einen Vorsprung auf, der zur Führung in eine Ausnehmung am zweiten Anschlusselement oder am Gehäuse des Atemluftbefeuchters eingreift. Die Verbindung zwischen Beatmungsschlauch und Flüssigkeitsbehälter kann damit in einer vorbestimmten Art und Weise erfolgen, die durch die Führungselemente festgelegt ist. Es ist dabei möglich, dass auf den entsprechenden Elementen mehrere Führungen angeordnet sind. Als besonders wirksam und ausreichend hat es sich jedoch erwiesen, wenn genau ein Führungselement pro Verbindungssystem vorgesehen ist.
Es ist weiter bevorzugt, dass das erste und das zweite elektrische Kontaktelement jeweils eine Mehrzahl von elektrischen Kontakten aufweisen. Hierbei ist es zweckmäßig, dass sich die Anzahl der elektrischen Kontakte des ersten und des zweiten Kontaktelements entsprechen. Es hat sich herausgestellt, das sechs elektrische Kontakte pro elektrischem Kontaktelement ausreichende Möglichkeiten bieten, um beispielsweise Heizleistung, Daten- oder Messinformationen elektrisch über das Beatmungsschlauchsystem zu übertragen.
Mit besonderem Vorteil ist bei korrekt hergestellter pneumatischer und elektrischer Verbindung zwischen dem ersten und dem zweiten Anschlusselement ein elektrischer Signalweg geschlossen. Damit kann das Ereignis der korrekten vollständigen Verbindung beispielsweise über eine Anzeigevorrichtung auf dem Atemluftbefeuchter angezeigt werden. Alternativ sind andere optische wie auch akustische Signale möglich.
Bevorzugt weist das erste elektrische Kontaktelement Ausnehmungen auf, in die dazu passende Vorsprünge des zweiten elektrischen Kontaktelements eingreifen, um den elektrischen Kontakt zu bewirken. Die Ausnehmungen bzw. Vorsprünge liegen dann nebeneinander, sind beispielsweise von zwei Richtungen her zugänglich, d. h. von oben und von der Seite, und können verschiedene Materialien und Dimensionen aufweisen je nach zu übertragender Leistung oder Stabilität der elektrischen Kontaktverbindung. In ähnlicher Weise können die elektrischen Kontaktelemente mit einer oder mehreren Federn versehen sein. Vorteilhaft ist es, wenn der Beatmungsschlauch eine Mehrzahl von elektrischen Leitungen aufweist, wobei vorzugsweise (mindestens) eine als Heizdrahtschleife bzw. Heizdrahtwendel, Stromzuführungsleitung, Messleitung oder Datenleitung ausgebildet ist. Somit können auch Daten oder Signale nicht nur im Atemluftbefeuchter ausgewertet werden, sondern beispielsweise auch vom Patienten zum Beatmungsgerät oder umgekehrt durchgeschleift werden.

Weiterhin beschrieben wird ein Beatmungssystem umfassend ein Beatmungsgerät, einen ersten Inspirationsschlauch, einen zweiten Inspirationsschlauch, einen Atemluftbefeuchter und zwei Verbindungssysteme wie oben beschrieben, wobei die beiden Verbindungssysteme den ersten bzw. zweiten Inspirationsschlauch mit dem Atemluftbefeuchter verbinden.

Mit besonderem Vorteil sind der erste Inspirationsschlauch und der zweite Inspirationsschlauch wahlweise beim ersten oder zweiten Verbindungssystem angeschlossen, wobei die Flussrichtung des Atemgases über die elektrischen Kontaktelemente erkennbar ist. Der Atemluftbefeuchter ist dadurch flexibel einsetzbar, denn er erkennt, wo der zum Beatmungsgerät, d. h. der Quelle des Atemgases, führende Inspirationsschlauch angeordnet ist und legt dadurch die Richtung des Atemgasflusses durch den Atemluftbefeuchter fest. Bedienungsfehler des Benutzers werden dadurch weiter reduziert, da ein verkehrtes Anschließen der Inspirationsschläuche nicht mehr zu einer Fehlfunktion führt.

Die Erfindung soll nachfolgend anhand von Ausführungsbeispielen unter Bezugnahme auf die beigefügten Figuren erläutert werden, in denen:
- Fig. 1: eine schematische Darstellung einer Ausführungsform des Beatmungssystems der vorliegenden Erfindung zeigt;
- Fig. 2: eine perspektivische Ansicht eines Flüssigkeitsbehälters mit Teilen des erfindungsgemäßen Verbindungssystems gemäß einer bevorzugten Ausführungsform zeigt;
- Fig. 3: eine perspektivische Ansicht eines Inspirationsschlauchs mit Teilen des erfindungsgemäßen Verbindungssystems gemäß der bevorzugten Ausführungsform zeigt;
- Fig. 4: eine perspektivische Ansicht eines Gehäuses eines Atemluftbefeuchters mit Teilen des erfindungsgemäßen Verbindungssystems gemäß der bevorzugten Ausführungsform zeigt;
- Fig. 5: einen vergrößerten Ausschnitt aus Fig. 4 zeigt; und
- Fig. 6: eine perspektivische Ansicht eines Atemluftbefeuchters mit zwei erfindungsgemäßen Verbindungssystemen gemäß der bevorzugten Ausführungsform zeigt.

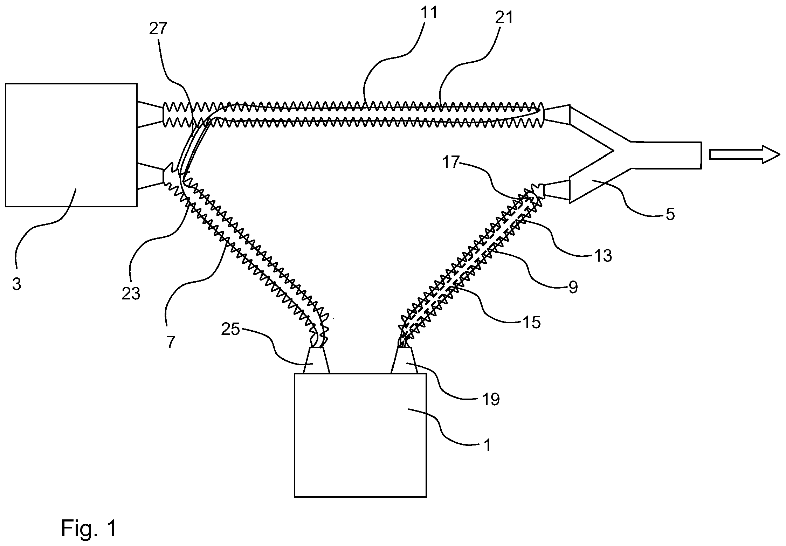

Fig. 1 zeigt in schematischer Darstellung eine bevorzugte Ausführungsform eines Beatmungssystems mit dem erfindungsgemäßen Verbindungssystem. Ein Atemluftbefeuchter 1 ist zwischen einem Beatmungsgerät 3 und einem Y-Stück 5 angeordnet und mit diesen über einen ersten Inspirationsschlauch 7 bzw. einen zweiten Inspirationsschlauch 9 verbunden. Das einfach ausgebildete Ende des Y-Stücks 5 zeigt mit dem dargestellten Pfeil in Richtung des zu beatmenden Patienten. Schließlich ist ein Exspirationsschlauch 11 zwischen dem Beatmungsgerät 3 und dem verbleibenden Ende des Y-Stücks 5 angeordnet.

Trockenes Atemgas wird beispielsweise über ein (nicht dargestelltes) Gebläse im Beatmungsgerät 3 erzeugt und verlässt dieses über den ersten Inspirationsschlauch 7 in Richtung des Atemluftbefeuchters 1. Dort wird das Atemgas in bekannter Art und Weise in einen (in Fig. 1 nicht dargestellten) Flüssigkeitsbehälter geleitet, wo es durch die erhitzte Flüssigkeit erwärmt und befeuchtet wird. Über den zweiten Inspirationsschlauch 9 verlässt das erwärmte und befeuchtete Atemgas den Atemluftbefeuchter 1 wieder und wird dem Patienten über das Y-Stück 5 zugeführt.

Entsprechend einem über das Beatmungsgerät 3 gesteuerten Atemzyklus fließt die verbrauchte Atemluft vom Patienten zurück und tritt am Y-Stück 5 in den Exspirationsschlauch 11 ein und wird wieder dem Beatmungsgerät 3 zugeführt.

In die Wand des zweiten Inspirationsschlauches 9 ist ein Heizdraht 13 integriert, der beispielsweise spiralförmig als Heizwendel ausgebildet ist. Weiterhin in den zweiten Inspirationsschlauch 9 integriert ist eine elektrische Messleitung 15, die das Signal von einem am Y-Stück 5 nahen Ende angeordneten Temperatursensor 17 zur (nicht dargestellten) Steuereinrichtung des Atemluftbefeuchters 1 überträgt. Der Ort des Temperatursensors 17 ist so gewählt, dass er möglichst nahe am Patienten liegt und doch noch Teil des möglichst einfach austauschbaren Beatmungsschlauchsystems ist.

Die pneumatische und elektrische Verbindung des zweiten Inspirationsschlauchs 9, d. h. der Einatemgasverbindung sowie des Heizdrahts 13 und der Messleitung 15, mit dem Atemluftbefeuchter 1 erfolgt über ein erstes Verbindungssystem 19.

Auch der Exspirationsschlauch 11 weist eine Schlauchheizung in Form des Heizdrahtes 21 auf, der ebenfalls als Heizwendel spiralförmig ausgebildet ist. Der Grund für die Beheizung des Exspirationsschlauches 11 ist, dass vermieden werden soll, dass zurückströmendes Atemgas im Exspirationsschlauch 11 kondensiert und beispielsweise als verunreinigte Flüssigkeit über das Y-Stück 5 wieder zurück zum Patienten fließt. Die Schlauchheizung des Exspirationsschlauches 11 kann durchgängig oder aber abschnittsweise ausgebildet sein. Mit Strom versorgt wird der Heizdraht 21 über eine Stromversorgungsleitung 23, die ausgehend vom Atemluftbefeuchter 1 über ein zweites Verbindungssystem 25 und den ersten Inspirationsschlauch 7 sowie ein Verbindungselement 27 zum Exspirationsschlauch 11 geführt ist. Das zweite Verbindungssystem 25 stellt demnach die pneumatische und elektrische Verbindung des ersten Inspirationsschlauchs 7, d. h. der Einatemgasverbindung sowie der Stromversorgungsleitung 23 und dem Atemluftbefeuchter 1 her.

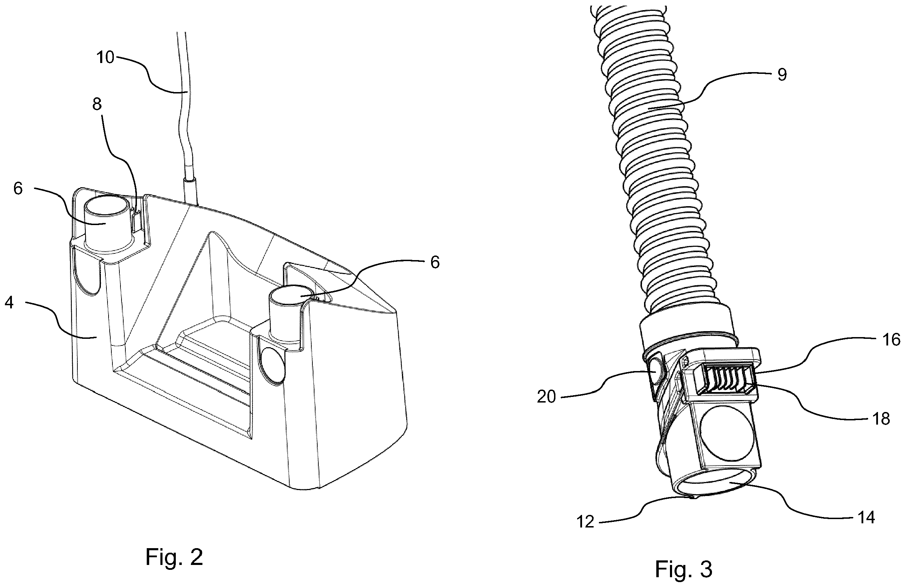

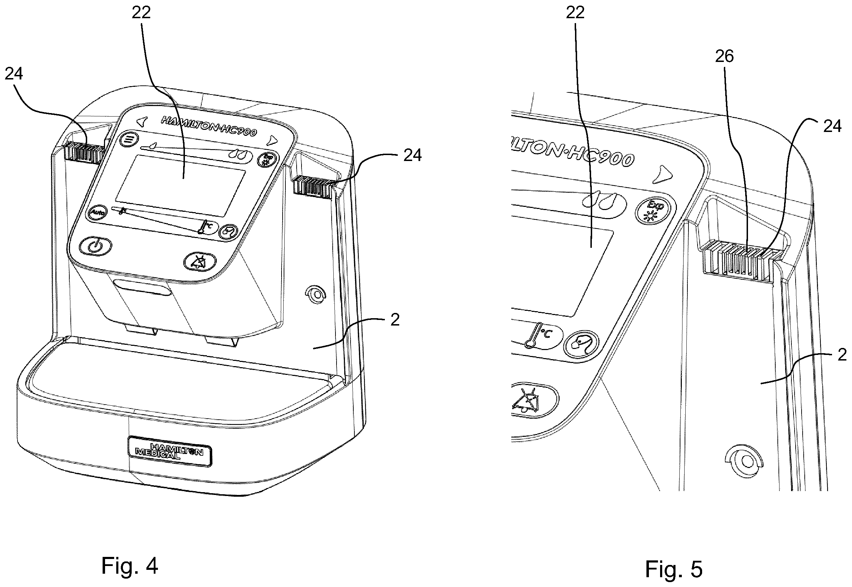

Fig. 2 zeigt eine perspektivische Ansicht eines Flüssigkeitsbehälters 4 mit Teilen des erfindungsgemäßen Verbindungssystems gemäß einer bevorzugten Ausführungsform der Erfindung. Der Flüssigkeitsbehälter 4 weist zwei erste Anschlusselemente 6 auf, die außen an seinem oberen Abschnitt angeordnet sind. Zwischen den beiden Anschlusselementen 6 ist ein Einschnitt ausgebildet, der passend zu dem (in Fig. 2 nicht dargestellten) Gehäusevorsprung ausgebildet ist, um den herum er in das Gehäuse eingeschoben werden kann, wie in den Fig. 4 bis 7 gezeigt ist. Die ersten Anschlusselemente 6 sind als hervorstehende Rohrstutzen mit kreisförmigem Querschnitt ausgebildet, sie können aber auch einen ellipsenförmigen oder polygonalen oder auch rechteckigen Querschnitt aufweisen.

In einem geringen Abstand zu den ersten Anschlusselementen 6, d. h. ausreichend für das Einschieben des zweiten Anschlusselements 14, ist jeweils eine Führung 8 angeordnet, die dazu dient, das zweite Anschlusselement 14 in einer korrekten axialen Ausrichtung auf das erste Anschlusselement 6 aufzuschieben. Weiterhin ist erkennbar, dass die ersten Anschlusselemente 6 nicht über den Rand des Flüssigkeitsbehälters 4 hinausragen. Dies hat den Vorteil, dass sie weniger leicht beschädigt werden können, wenn der Flüssigkeitsbehälter 4 auf die Rück- oder Oberseite fällt. Ein Nachfüllschlauch 10 ist über ein geeignetes Anschlussstück mit dem Flüssigkeitsbehälter 4 verbunden. Da das Innenvolumen des Flüssigkeitsbehälters 4 begrenzt ist, wird bei Unterschreitung eines bestimmten Wasserniveaus im Inneren des Flüssigkeitsbehälters 4 Wasser über den Nachfüllschlauch 10 nachgeführt. Das Material des Flüssigkeitsbehälters 4 ist ein geeigneter transparenter Kunststoff wie z. B. Polyethylen (PE) oder Polypropylen (PP). Es können auch andere geeignete Kunststoffmaterialien verwendet werden.

Fig. 3 zeigt eine perspektivische Ansicht eines Inspirationsschlauchs 9 mit Teilen des erfindungsgemäßen Verbindungssystems gemäß der bevorzugten Ausführungsform der vorliegenden Erfindung. Das eine Ende des ersten Inspirationsschlauchs 9 bildet ein Anschlussstück, dessen schlauchfernes Ende von dem zweiten Anschlusselement 14 gebildet ist. Weiterhin weist das Anschlussstück ein zweites elektrisches Kontaktelement 16 auf, das seitlich an dem Anschlussstück angeordnet ist. Es ist mit elektrischen Kontakten ausgestattet, die als Vorsprünge 18 ausgebildet sind. Ebenfalls seitlich, und zwar um etwa 90° zu dem zweiten elektrischen Kontaktelement 16 versetzt, weist das Anschlussstück Griffelemente 20 auf, die als Angriffspunkte zum Lösen der Verbindung dienen. Auf der dem zweiten elektrischen Kontaktelement 16 gegenüber liegenden Seite weist das zweite Anschlusselement 14 einen länglichen Vorsprung 12 auf der Außenseite auf, der in Axialrichtung verläuft und dazu geeignet ist, in die Führung 8 einzugreifen, die in Fig. 2 gezeigt ist. Zur optisch und haptisch ansprechenden Ausgestaltung weist das Anschlussstück eine abgeschrägte Schürze auf, die im eingebauten Zustand des Verbindungssystems auf dem Flüssigkeitsbehälter 4 für eine im Wesentlichen durchgängige Deckelfläche sorgen soll.

In der bevorzugten Ausführungsform wird das zweite Anschlusselement 14 vollständig auf das erste Anschlusselement 6 entlang der Führung aufgeschoben, d. h. in einer Presspassung bis ganz nach unten auf den Anschlag des Flüssigkeitsbehälters 4. Alternativ kann ein Rastmechanismus zwischen den beiden Anschlusselementen 6, 14 angeordnet sein, der beispielsweise durch Drücke der Griffelemente 20 wieder gelöst werden kann.

Fig. 4 zeigt eine perspektivische Ansicht eines Gehäuses 2 eines Atemluftbefeuchters 1 mit Teilen des erfindungsgemäßen Verbindungssystems gemäß der bevorzugten Ausführungsform der vorliegenden Erfindung. Das Gehäuse 2 ist im Wesentlichen L-förmig ausgebildet mit einem waagerechten Schenkel und einem senkrechten Schenkel und weist auf dem waagerechten Schenkel eine Heizplatte auf, die dazu dient, die Flüssigkeit in dem (in Fig. 4 nicht dargestellten) Flüssigkeitsbehälter 4 zu erwärmen. Am freien Ende des senkrechten Schenkels weist das Gehäuse 2 im mittleren Bereich einen schrägen vorsprungartigen Abschnitt auf, der eine Benutzerschnittstelle 22 umfasst, die mit einer Anzeige und Bedienelementen versehen ist. Das Innere des Gehäuses 2 umfasst unter anderem eine Steuereinrichtung, die beispielsweise geeignet ist, die Heizleistung der Heizplatte entsprechend der von verschiedenen Temperatursensoren empfangenen Signale zu regeln. Darüber hinaus kann das Gehäuse 2 noch weitere funktionale Elemente umfassen, die nicht zum Umfang der vorliegenden Erfindung gehören. Seitlich am senkrechten Abschnitt des Gehäuses 2 in seinem oberen Bereich sind erste elektrische Kontaktelemente 24 ausgebildet, die zu den zweiten elektrischen Kontaktelementen 16 des in Fig. 3 dargestellten Anschlussstücks passen.

Fig. 5 zeigt in vergrößerter Darstellung den oberen rechten Bereich aus Fig. 4. Man erkennt, dass das erste elektrische Kontaktelement 24 am oberen Ende einer im wesentlichen senkrechten Gehäusewand angeordnet ist und in eine Aussparung hinein versetzt ist, wobei die elektrischen Kontakte durch Ausnehmungen 26 gebildet sind, die exakt zu den Vorsprüngen 18 des zweiten elektrischen Kontaktelements 16 (siehe Fig. 3) passen. Aus einer Zusammenschau der elektrischen Kontaktelemente 16 und 24 aus den Fig. 3 bzw. 5 lässt sich entnehmen, dass die elektrische Verbindung zwischen dem Anschlussstück aus Fig. 3 und dem Gehäuse 2 aus Fig. 4 sowohl durch Einsetzen von oben als auch durch seitliches Einschieben in horizontaler Richtung erfolgen kann.

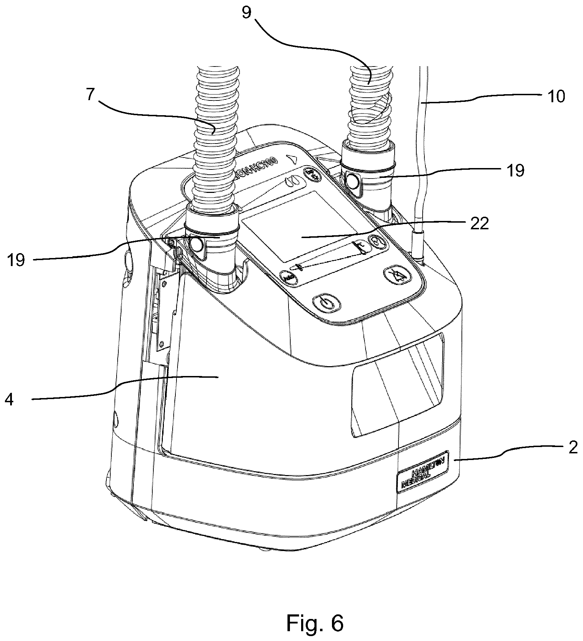

Fig. 6 zeigt eine perspektivische Ansicht eines Atemluftbefeuchters mit zwei erfindungsgemäßen Verbindungssystemen gemäß der bevorzugten Ausführungsform der vorliegenden Erfindung. Der Gegenstand der Fig. 6 ergibt sich aus der Kombination des Flüssigkeitsbehälters 4 aus Fig. 2 mit dem Schlauchanschlussstück aus Fig. 3 (in zweifacher Ausführung) und dem in Fig. 4 dargestellten Gehäuse 2. Der Flüssigkeitsbehälter 4 ist dabei waagerecht in den freigestellten Bereich des Gehäuses 2 eingeschoben, so dass der Bodenabschnitt des Flüssigkeitsbehälters 4 im Wesentlichen auf dem unteren Abschnitt des Gehäuses 2 aufliegt, in dem sich die Heizplatte des Atemluftbefeuchters 1 befindet. Auf die beiden ersten Anschlusselemente 6 wird jeweils ein Anschlussstück aufgeschoben, dass sich am Ende des ersten bzw. zweiten Inspirationsschlauches 7, 9 befindet.

Das Aufsetzen des zweiten Anschlusselements 14 auf das Gegenstück, nämlich das erste Anschlusselement 6, entlang des Vorsprungs 12 innerhalb der Führung 8 bewirkt bei aus dem Gehäuse 2 herausgenommenem Flüssigkeitsbehälter 4 lediglich die pneumatische Verbindung zwischen Schlauch und Flüssigkeitsbehälter 4. Die elektrische Verbindung zwischen den elektrischen Mess-, Heiz- oder Datenleitungen in den Schläuchen 7, 9 mit dem Gehäuse 2 kann auf zwei unterschiedliche Art und Weisen erfolgen.

Die erste Möglichkeit ist, den Flüssigkeitsbehälter 4 in das Gehäuse 2 einzusetzen und dann die Schläuche 7, 9 mittels des Verbindungssystems aufzusetzen und so die pneumatische und elektrische Verbindung gleichzeitig herzustellen. Die Verbindungsrichtung ist in diesem Fall senkrecht von oben in axialer Richtung der Schlauchenden.
Die zweite Möglichkeit wird bei den als Einmal-/Wegwerfartikeln ausgebildeten Beatmungsschlauchsystemen jedoch häufiger anwendbar sein, denn der Flüssigkeitsbehälter 4 wird in üblicher Weise zusammen mit dem kompletten Beatmungsschlauchsystem, d. h. dem ersten Inspirationsschlauch 7, dem zweiten Inspirationsschlauch 9 sowie dem Expirationsschlauch 11 und gegebenenfalls dem Y-Stück 5 (siehe Fig. 1) als Einheit verbunden angeliefert, so dass das Bedienpersonal möglichst wenige Verbindungsschritte auszuführen hat. Zur Verbindung des Flüssigkeitsbehälters 4 mit dem Gehäuse 2 des Atemluftbefeuchters 1 wird der Flüssigkeitsbehälter in horizontaler Richtung, d. h. senkrecht zur Schlauchrichtung und zur Verbindungsrichtung der pneumatischen Leitungen in das Gehäuse 2 eingesetzt. Flüssigkeitsbehälter 4 und Gehäuse 2 sind derart ausgebildet, dass der Flüssigkeitsbehälter 4 über einen Rastmechanismus für den Benutzer spürbar einrastet. In Fig. 6 ist die eingerastete Endposition des Flüssigkeitsbehälters 4 im Gehäuse 2 dargestellt, und beim Einrasten des Flüssigkeitsbehälters 4 in das Gehäuse 2 wird auch die elektrische Verbindung zwischen dem zweiten elektrischen Kontaktelement 16 der Anschlusstücke 7, 9 mit den entsprechenden Gegenstücken der ersten elektrischen Kontaktelemente 24 bewirkt. Die Steuereinrichtung kann bei einer vollständig bewirkten elektrischen und pneumatischen Verbindung ein akustisches Signal ausgeben oder auf der Benutzerschnittstelle 22 anzeigen.
Mit dem Gegenstand der vorliegenden Erfindung wurde ein Verbindungssystem zum Anschließen eines Beatmungsschlauches an einen Atemluftbefeuchters bereit gestellt, das flexible Anschlussmöglichkeiten für die elektrischen Kontaktelemente bietet und somit eine verbesserte Konstruktion sowie ein ansprechendes Design des Atemluftbefeuchters ermöglicht. Es sei an dieser Stelle noch angemerkt, dass das Verbindungssystem vom Prinzip her auch für die Verbindung des zweiten Endes des ersten Inspirationsschlauchs mit dem Beatmungsgerät oder auch für die Verbindung des Exspirationsschlauchs 11 mit dem Beatmungsgerät 3 verwendet werden kann.

## Patentansprüche

1. Verbindungssystem zum Anschließen eines Beatmungsschlauches (7, 9) an einen Atemluftbefeuchter (1) in Form eines Flüssigkeitsbehälter-Beatmungsschlauchsystem-Kits, mit
einem Beatmungsschlauch (7, 9), der eine elektrische Leitung aufweist,
einem Flüssigkeitsbehälter (4), der in ein Gehäuse (2) des Atemluftbefeuchters (1) einsetzbar ist,
einem ersten Anschlusselement (6), das an dem Flüssigkeitsbehälter (4) angeordnet ist, und
einem zweiten Anschlusselement (14), das mit dem Beatmungsschlauch (7, 9) verbunden ist und ein elektrisches Kontaktelement (16) aufweist, das mit der elektrischen Leitung des Beatmungsschlauches (7, 9) verbunden ist,
wobei das erste Anschlusselement (6) und das zweite Anschlusselement (14) entlang einer ersten Verbindungsrichtung miteinander verbindbar sind, so dass eine pneumatische Verbindung des Beatmungsschlauches (7, 9) mit dem Flüssigkeitsbehälter (4) bewirkt wird,
**dadurch gekennzeichnet, dass**
die elektrische Verbindung zwischen dem elektrischen Kontaktelement (16) des zweiten Anschlusselements (14) und einem am Gehäuse (2) befindlichen elektrischen Kontaktelement (24) sowohl in der ersten Verbindungsrichtung als auch senkrecht zur ersten Verbindungsrichtung herstellbar ist.

2. Verbindungssystem nach Anspruch 1, **dadurch gekennzeichnet, dass** das erste und das zweite Anschlusselement (6, 14) rohrförmig ausgebildet sind und einen kreisförmigen Querschnitt aufweisen, und das erste und das zweite Anschlusselement (6, 14) entlang der ersten Verbindungsrichtung axial ineinander einschiebbar sind.

3. Verbindungssystem nach Anspruch 2, **dadurch gekennzeichnet, dass** das zweite Anschlusselement (14) außen einen Vorsprung (12) aufweist, der zur Führung in eine Ausnehmung (8) am ersten Anschlusselement (6) des Atemluftbefeuchters (1) eingreift.

4. Verbindungssystem nach einem der vorhergehenden Ansprüche, **dadurch gekennzeichnet, dass** bei korrekt hergestellter pneumatischer und elektrischer Verbindung zwischen dem ersten und dem zweiten Anschlusselement (6, 14) ein elektrischer Signalweg geschlossen ist.

5. Verbindungssystem nach einem der vorhergehenden Ansprüche, **dadurch gekennzeichnet, dass** der Beatmungsschlauch (7, 9) eine Mehrzahl von elektrischen Leitungen aufweist.

6. Verbindungssystem nach einem der vorhergehenden Ansprüche, **dadurch gekennzeichnet, dass** eine elektrische Leitung als Heizdrahtschleife (13, 21) bzw. Heizdrahtwendel, Stromzuführungsleitung, Messleitung oder Datenleitung ausgebildet ist.

7. Beatmungssystem umfassend ein Beatmungsgerät (3), einen Atemluftbefeuchter (1) mit einem Gehäuse (2) und ein Verbindungssystem nach einem der vorhergehenden Ansprüche, **dadurch gekennzeichnet, dass** die elektrischen Kontaktelemente (24, 16) jeweils eine Mehrzahl von elektrischen Kontakten aufweisen, vorzugsweise sechs elektrische Kontakte.

8. Beatmungssystem nach Anspruch 5, **dadurch gekennzeichnet, dass** das elektrische Kontaktelement (24) des Gehäuses (2) Ausnehmungen (26) aufweist, in die dazu passende Vorsprünge (18) des elektrischen Kontaktelements (16) des zweiten Anschlusselements (14) eingreifen, um den elektrischen Kontakt zu bewirken.

## Claims

1. Connection system for attaching a breathing tube (7, 9) to a respiratory humidifier (1) in the form of a fluid container breathing tube system kit, with
a breathing tube (7, 9) which has an electric lead,
a fluid container (4) which can be inserted into a housing (2) of the respiratory humidifier (1),
a first attachment element (6) which is arranged on the fluid container (4), and
a second attachment element (14) which is connected to the breathing tube (7, 9) and has an electrical contact element (16) which is connected to the electric lead of the breathing tube (7, 9),
wherein the first attachment element (6) and the second attachment element (14) can be connected to one another along a first connecting direction, so that a pneumatic connection of the breathing tube (7, 9) with the fluid container (4) is actuated,
**characterised in that**
the electric connection between the electrical contact element (16) of the second attachment element (14) and an electrical contact element (24) located on the housing (2) can be produced both in the first connecting direction and also perpendicular to the first connecting direction.

2. Connection system according to claim 1, **characterised in that** the first and the second attachment element (6, 14) are of a tubular design and have a circular cross-section, and the first and the second attachment element (6, 14) can be slid axially into one another along the first connecting direction.

3. Connection system according to claim 2, **characterised in that** the second attachment element (14) has on the outside a projection (12) which engages for guidance into a recess (8) on the first attachment element (6) of the respiratory humidifier (1).

4. Connection system according to one of the preceding claims **characterised in that** an electrical signal path is closed in the event of a correctly produced pneumatic and electrical connection between the first and the second attachment elements (6, 14).

5. Connection system according to one of the preceding claims, **characterised in that** the breathing tube (7, 9) has a plurality of electric leads.

6. Connection system according to one of the preceding claims, **characterised in that** an electric lead is formed as the heating wire loop (13, 21) or heating wire coil, current supply lead, measuring lead or data lead respectively.

7. Breathing system comprising a breathing apparatus (3), a respiratory humidifier (1) with a housing (2) and a connection system according to one of the preceding claims, **characterised in that** the electrical contact elements (24, 16) each have a plurality of electrical contacts, preferably six electrical contacts.

8. Breathing system according to claim 5, **characterised in that** the electrical contact element (24) of the housing (2) has recesses (26) into which correspondingly fitting projections (18) of the electrical contact element (16) of the second attachment element (14) engage in order to make the electrical contact.

## Revendications

1. Système de raccordement permettant de raccorder un tuyau respiratoire (7, 9) à un humidificateur d'air inhalé (1) se présentant sous forme d'un kit de réservoir à liquide et de système de tuyau respiratoire, comprenant
un tuyau respiratoire (7, 9), qui comporte une première ligne électrique,
un réservoir à liquide (4), qui peut être inséré dans un boîtier (2) de l'humidificateur d'air inhalé (1),
un premier élément de raccordement (6), qui est agencé sur le réservoir à liquide (4), et
un deuxième élément de raccordement (14), qui est raccordé au tuyau respiratoire (7, 9) et comporte un élément de contact électrique (16) qui est raccordé à la ligne électrique du tuyau respiratoire (7, 9),
le premier élément de raccordement (6) et le deuxième élément de raccordement (14) pouvant être raccordés l'un à l'autre le long d'une première direction de raccordement, de sorte qu'une liaison pneumatique entre le tuyau respiratoire (7, 9) et le réservoir à liquide (4) est réalisée,
**caractérisé en ce que**
la liaison électrique entre l'élément de contact électrique (16) du deuxième élément de raccordement (14) et un élément de contact électrique (24) situé sur le boîtier (2) peut être établie aussi bien dans la première direction de raccordement que perpendiculairement à la première direction de raccordement.

2. Système de raccordement selon la revendication 1, **caractérisé en ce que** le premier et le deuxième élément de raccordement (6, 14) sont tubulaires et présentent une section transversale circulaire, et le premier et le deuxième élément de raccordement (6, 14) peuvent être insérés axialement l'un dans l'autre le long de la première direction de raccordement.

3. Système de raccordement selon la revendication 2, **caractérisé en ce que** le deuxième élément de raccordement (14) présente à l'extérieur une partie saillante (12) qui, pour le guidage, s'insère dans un évidement (8) sur le premier élément de raccordement (6) de l'humidificateur d'air inhalé (1).

4. Système de raccordement selon l'une quelconque des revendications précédentes, **caractérisé en ce que**, lorsqu'une liaison pneumatique et électrique est correctement établie entre le premier et le deuxième élément de raccordement (6, 14), un trajet de signal électrique est fermé.

5. Système de raccordement selon l'une quelconque des revendications précédentes, **caractérisé en ce que** le tuyau respiratoire (7, 9) comprend une pluralité de lignes électriques.

6. Système de raccordement selon l'une quelconque des revendications précédentes, **caractérisé en ce qu'**une ligne électrique est réalisée sous la forme d'une boucle de filament chauffant (13, 21) ou d'un filament chauffant hélicoïdal, d'une ligne d'alimentation en courant, d'une ligne de mesure ou d'une ligne de données.

7. Système respiratoire comprenant un appareil respiratoire (3), un humidificateur d'air inhalé (1) comportant un boîtier (2) et un système de raccordement selon l'une quelconque des revendications précédentes, **caractérisé en ce que** les éléments de contact électriques (24, 16) présentent chacun une pluralité de contacts électriques, de préférence six contacts électriques.

8. Système respiratoire selon la revendication 5, **caractérisé en ce que** l'élément de contact électrique (24) du boîtier (2) présente des évidements (26) dans lesquels s'insèrent des parties saillantes (18), adaptées à ces derniers, de l'élément de contact électrique (16) du deuxième élément de raccordement (14), afin de réaliser le contact électrique.
